Europäisches Patentamt

European Patent Office

Office européen des brevets

(19)

(11) Publication number : **0 327 086 B1**

(12) **EUROPEAN PATENT SPECIFICATION**

(45) Date of publication of patent specification :
06.05.92 Bulletin 92/19

(51) Int. Cl.⁵ : **A61K 9/54**

(21) Application number : **89101806.1**

(22) Date of filing : **02.02.89**

(54) **Multi-layer granule.**

(30) Priority : **03.02.88 JP 23215/88**

(43) Date of publication of application :
**09.08.89 Bulletin 89/32**

(45) Publication of the grant of the patent :
**06.05.92 Bulletin 92/19**

(84) Designated Contracting States :
**AT BE CH DE ES FR GB IT LI NL SE**

(56) References cited :
**EP-A- 0 074 584**
**EP-A- 0 220 670**
**US-H- 861 008**

(73) Proprietor : **Eisai Co., Ltd.**
**6-10, Koishikawa 4-chome Bunkyo-ku**
**Tokyo (JP)**

(72) Inventor : **Aoki, Shigeru**
**Espoa Ginan 1-A, 98 Tokudanishi 2-chome**
**Ginancho**
**Hashima-gun Gifu (JP)**
Inventor : **Uesugi, Keizo**
**157, Aza Miyanishi Ohaza Minamiyana**
**Fusocho**
**Niwa-gun Aichi (JP)**
Inventor : **Sakashita, Shigeru**
**713, Kitaoyobi Kasamatsucho**
**Hashima-gun Gifu (JP)**
Inventor : **Kasai, Masayoshi**
**5-156, Tsutsujigaoka**
**Kakamigahara-shi Gifu (JP)**
Inventor : **Kayano, Masanori**
**9-20, Idogi 1-chome**
**Ageo-shi Saitama (JP)**

(74) Representative : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

EP 0 327 086 B1

## Description

This invention relates to a multi-layer granule comprising a slow release layer and a rapid release layer outwardly surrounding the slow release layer, and more particularly to a multi-layer granule into which slow release part and rapid release part are integrated in such a way that concerned effective component(s) can be released over a thereby-prolonged time.

## The Prior Art

In treatment with drug, it is frequently required to sustain adequate therapeutic concentration of it in the living body over a necessary time. Sustained action preparations have been designed for this purpose. These are divided into a number of types and devised from various aspects. In some cases, preparations are useful which comprise rapid and slow release parts so that in the living body, effective blood concentration may be early achieved and maintained. As examples of such preparations are mentioned hard capsule type comprising slow and rapid release granules previously prepared, respectively, mixed and charged together in a hard capsule, and multi-layer granule type disclosed in a Japanese laid-open patent No.103012/1987 applied by the present inventors.

EP-A-0 220 670 discloses a multi-layer granule having an inner slow-releasing layer and an outer rapid-releasing layer. These two layers are separated by an intermediate layer formed of a metal stearate as an essential ingredient, and optionally including a film-forming material such as ethyl cellulose or hydroxypropylmethyl cellulose.

EP-A-0 074 584 discloses a prolonged action multiple-layer tablet. This comprises a first layer made of an intimate mixture of a water-insoluble wax formed from certain oils, and a disintegrator such as hydroxypropyl cellulose. A second layer is provided comprising similar constituents together with an active pharmaceutical ingredient. A quick release layer containing the active ingredient may be formed on either of the previously mentioned layers.

The above-mentioned conventional sustained release drugs however have not fully met the requirements. There are difficulties with slow release, for instance, some can be dissolved with remarkable variation dependent on pH of solvent (in vitro), and some which utilize a low melting point wax system for the purpose of slow release may lose the slow release characteristic during the stock at high temperature (45°C).

## Summary of the Invention

Under the circumstances, the inventors studied in search of base or vehicle of drug unaffected substantially by pH of solvent and permitting the slow release characteristic to remain unaffected during the high temperature stock, and as a result hardened oil has been found out to be adequate for such base. Besides it has been discovered that the membranous layer composed of sucrose-fatty acid ester, talc, and ethyl cellulose was associated with a lag time of at least 3 hours while the incorporation of hydroxypropyl cellulose or methyl cellulose as water-path forming agent resulted in substantially no lag time. Thus the present invention has been accomplished.

It therefore is an object of the invention to provide a multi-layer granule comprising a slow release layer, a rapid release layer outwardly surrounding the slow release layer, and an intermediate membranous layer therebetween made essentially from a hardened oil and hydroxypropyl cellulose or methyl cellulose.

The invention provides a multi-layered granule comprising an inner, slow-releasing layer, an outer, rapid-releasing layer and an intermediate layer, provided between said slow-releasing layer and said rapid-releasing layer, which intermediate layer comprises 20-90% by weight of a hardened oil and 1-10% by weight of hydroxypropyl cellulose or methyl cellulose.

From the practical point of view, the granule of the invention comprises a core, an inner, slow-releasing layer comprising a pharmacologically effective ingredient, an outer, rapid-releasing layer comprising a pharmacologically effective ingredient and an intermediate layer, provided between said slow-releasing layer and said rapid-releasing layer, which intermediate layer comprises 20-90% by weight of a hardened oil and 1-10% by weight of hydroxypropyl cellulose or methyl cellulose.

The intermediate layer may include a balance comprising a third component as described below.

Examples of hardened oils suitable for use in the present invention include hardened castor, rape and soybean oils. The membranous layer between the slow and rapid release layers is preferred to contain hardened oil within the range of 20 to 80% by weight based on the total weight of the membranous layer.

Multi-layer granules according to the invention can be produced with slow release granules as starting material or with a granular seed. Suitable granular seeds (referred to as NPS) include generally-available granules formed of white sugar or white sugar-corn starch mixture. The starting granule is, for instance, a pellet made in the process comprising kneading a mixture of medicine to be slowly-released and other ingredients together with a binder, and projecting the resultant. The present invention is not limited to these processes. Seed may be used in a conventional hay to form slow release layer surrounding it.

Over the slow release layer is formed a membranous layer to be an intermediate layer comprising essentially a hardened oil and hydroxypropyl cellulose or methyl cellulose by coating. The coating may be conducted by spraying a liquid preparation to be formed into the membranous layer while the subjects to be coated are flowing and rolling. The liquid preparation is prepared in the procedure consisting of mixing the above-mentioned essential ingredients with sucrose-fatty acid ester, talc, ethyl cellulose, and dissolving or dispersing the resulting mixture in a solvent such as ethyl alcohol.

Over the resulting granules having the thus-formed membranous layer, a rapid release layer is formed. This may be done by the same method as in forming the intermediate membranous layer.

The thus-obtained multi-layer granular drugs may be used alone or in combination.

[Examples]

The invention will be described more fully by way of examples and is not limited by the examples.

Example 1

NPS (2.87 kg), 28 to 32 mesh-sieved (0.495-0.589 mm), granules were used as seed. Phenylpropanolamine hydrochloride (840g), chlorpheniramine d-maleate (42g), veradonna alkaloid (7g), talc (290.5g), light silicic acid anhydride (70g), and hydroxypropyl cellulose (45.5g), were dissolved and dispersed in ethyl alcohol (3 liter), and the resulting preparation was gradually fed into a small dish-revolving granulator (CF granulator), where seed granules are allowed to be rolling, thus to form a layer (called R layer) over the seed granules, and dried at 45°C with drafting for 12 hours. The obtained granules were called R granules.

Subsequently another preparation which was prepared by dissolving and dispersing a hardened oil (176g), talc (88g), hydroxypropyl cellulose 9g, ethyl cellulose 7.5g, purified shellac (7.5g) in a 1 liter of ethyl alcohol was gradually sprayed on R granules (1.19 kg) that were allowed to be rolling on the CF granulator to form a new layer (called C layer) and then dried at 40°C with drafting for 12 hours. The obtained granules were termed C granules.

Subsequently, on the C granules (1428kg), while allowed to be rolling on the CF granulator, another preparation which was prepared by dissolving and dispersing phenylpropanolamine hydrochloride (320g), lysozyme chloride (460g), talc (200g), light silicic acid anhydride (50g), corn starch (442g), and hydroxypropyl cellulose 100g in 3.3 liter of ethyl alcohol was gradually sprayed to form further new layer (called I layer), and then dried at 40°C with drafting for 12 hours. The thus-obtained granules were termed I granules.

Besides on the C granules (1428kg), while allowed to be rolling on the CF granulator, another preparation which was prepared by dissolving and dispersing chlorpheniramine d-maleate (16g), caffeine (600g), dipotassium glycyrrhizate (300g), light silicic acid anhydride (50g), corn starch (686g), and hydroxypropyl cellulose (70g) in 3.3 liter of ethyl alcohol was gradually sprayed to form the new layer (called J layer), and then dried at 40°C with drafting for 12 hours. The thus-obtained granules were termed J granules.

I granules and J granules made as above-described were mixed in 1:1 (weight:weight) ratio, and thus the multi-layer granules according to the present invention were obtained.

By reference, the composition of the obtained multi-

```
Seed  NPS                                              82
R  layer:
      Phenylpropanolamine hydrochloride       24
      Chlorpheniramine d-maleate               1.2
      Veradonna alkaroid                       0.2
      Talc                                     8.3
      Light silicic acid anhydride             2
      Hydroxypropyl cellulose                  1.3
  C  layer
      Hardened oil                            17.6g,
      Talc                                     3.8
      Hydroxypropyl cellulose                  0.9g
      Ethyl cellulose                          0.75
      Purified shellac                         0.75
    I  layer
      Phenylpropanolamine hydrochloride       16
      Lysozyme chloride                       23
      Talc                                    10
      Light silicic acid anhydride             2.5
      Corn starch                             22.2
      Hydroxypropyl cellulose                  5
    J  layer
      Chlorpheniramine d-maleate               0.8
      Caffeine                                30
      Dipotassium glycyrrhizate               30
      Light silicic acid anhydride            10
      Corn starch                             31.8
      Hydroxypropyl cellulose                  3.5
```

layer granule, expressed in part by weight based on the entire weight of the granule, are as follows:

Example 2

This was conducted for making three different types of multi-layer granules (1), (2) and (3) according to the present invention in the same procedure as that of Example 1 except that the composition of C layer of each type is respectively as described in the following (expressed part by weight):

```
                                      (1)       (2)       (3)
  C  layer
      Hardened oil                    16.0      21.5      25.8
      Sucrose-fatty acid ester         1.5       2         2.4
      Talc                             3.8       5         6
      Hydroxypropyl cellulose          0.9       1.2       1.4
      Ethyl cellulose                  0.75      1         1.2
      Purified shellac                 0.75      1         1.2
```

Control 1

This was conducted for making another type of multi-layer granules in the same procedure as that of Example 1 except that C layer has a composition described in the following (expressed in part by weight):

```
C  layer                          :
      Hardened oil                          14.8
      Sucrose-fatty acid ester              8.4
      Talc                                  10.8
      Ethyl cellulose                        1.8
```

Example 3

This was conducted for making further three different types of multi-layer granules (4), (5) and (6) according to the present invention in the same procedure as that of Example 1 except that the composition of C layer of each type was respectively as described in the following (expressed in part by weight):

```
C  layer
                                    (4)      (5)      (6)
      Hardened oil                  7.4      7.4      7.4
      Sucrose-fatty acid ester      4.2      4.2      4.2
      Talc                          2.7      2.7      8.1
      Methyl cellulose              1.0      0.5       -
      Ethyl cellulose               0.9      0.9      0.9
```

Example 4

This was conducted for making further another type of multi-layer granules according to the present invention in the same procedure as that of Example 1 except that C layer had a composition described in the following (expressed in part by weight):

```
C  layer
      Hardened oil                          19.2
      Sucrose-fatty acid ester               1.8
      Talc                                   4.5
      Hydroxypropyl cellulose                1
      Ethyl cellulose                        0.9
      Purified shellac                       0.9
```

Advantages of the invention

The advantages of the invention will become apparent from the tests described in the following.

Test 1

Multi-layer granules of types obtained in Example 1 and Control 1 were used as samples. Each sample was place in a revolving basket (that in the Dissolution Test described in the Japanese Pharmacopoeia, 11 version), and immersed in the first solvent (the same Pharmacopoeia) for the first 2 hours. Thereafter dissolution proceeded by immersion in the second solvent (the same Pharmacopoeia). Dissolution percentages were determined by HPCL method.

The progresses in dissolution (represented by change in cumulative percentages with time) are summarized in Table 1.

### Table 1. Progresses in dissolution (cumulative percentage)

| Sample | Time (hours) | | | | | | |
|---|---|---|---|---|---|---|---|
| | 1 | 2 | 3 | 4 | 6 | 8 | 10 |
| Example 1 | 41.1 | 43.7 | 48.0 | 51.4 | 60.1 | 69.3 | 76.5 |
| Control 1 | 40.0 | 40.0 | 40.0 | 40.0 | 45.1 | 56.7 | 70.8 |

In Example 1, dissolution started substantially without lag time. On the other hand, Control 1 was done with lag time of at least 4 hours, and thus can not be regarded as good dissolution.

Test 2

Multi-layer granules of three types (C layer of each is (1), (2) and (3), respectively) according to the present invention obtained in Example 2 were used as samples. The changes in dissolution percentage with time were determined in the same way as in Test 1 as measured likewise by HPCL method.

The obtained results (as cumulative dilution percentages) were summarized in Table 2.

### Table 2. Progress in dissolution (cumulative percentage)

| Sample | Time (hours) | | | | | | |
|---|---|---|---|---|---|---|---|
| | 1 | 2 | 3 | 4 | 6 | 8 | 10 |
| (1) | 59.7 | 73.3 | 88.2 | 89.6 | 94.5 | 96.3 | 97.7 |
| (2) | 45.8 | 53.8 | 62.4 | 69.8 | 79.3 | 84.9 | 87.6 |
| (3) | 41.0 | 43.4 | 47.0 | 50.5 | 56.8 | 63.6 | 69.8 |

As apparent from Table 2, as to the multi-layer granules having a hardened oil-containing membrane layer according to the invention, the higher the content of hardened oil is, the release the slower becomes. Progress in dissolution therefore can be adjusted as desired by the modifying composition of the membrane layer.

Test 3

Multi-layer granules of three types (C layer of each is (4) and (5), respectively) according to the present invention, and control multi-layer granules (6) were used as samples. The progress in dissolution or change in dissolution percentage with time was determined in the same way as in Test 2.

The obtained results (as cumulative dilution percentages) were summarized in Table 3.

### Table 3. Progress in dissolution (cumulative percentage)

| Sample | Time (hours) | | | | | | |
|---|---|---|---|---|---|---|---|
| | 1 | 2 | 3 | 4 | 6 | 8 | 10 |
| (4) Invention | 43.3 | 51.8 | 65.8 | 77.3 | 84.9 | 94.3 | 99.0 |
| (5) Invention | 40.0 | 44.8 | 58.2 | 72.0 | 88.3 | 93.6 | 93.9 |
| (6) Control | 40.0 | 42.9 | 47.2 | 61.9 | 80.0 | 90.0 | 95.3 |

As apparent from Table 3, the of methyl cellulose contained in the membrane layer according to the present invention can cause lag time to reduce. The effect however is low compared with that of hydroxypropyl cellulose.

Test 4

Multi-layer granules of the type obtained in Example 4 according to the present invention were used as sample. Sample portions were maintained at room temperature for one month, at 40°C (RH 75%) for one month, and at 45°C for one month, respectively, and then underwent dissolution test in the same way as in Test 2.
The obtained results (as cumulative dilution percentages) were summarized in Table 4.

Table 4. Progress in dissolution (cumulative percentage)

| Stock conditions | Time (hours) | | | | | | |
|---|---|---|---|---|---|---|---|
| | 1 | 2 | 3 | 4 | 6 | 8 | 10 |
| Room temp. | 57.0 | 65.4 | 71.3 | 76.1 | 83.4 | 89.5 | 92.5 |
| 40°C, RH 75% | 53.6 | 63.3 | 70.8 | 75.4 | 81.9 | 86.7 | 90.2 |
| 45°C | 52.2 | 61.0 | 68.9 | 73.1 | 79.2 | 88.7 | 87.0 |

As revealed from Table 4, the membrane layer according to the invention keeps the protective effect on the release-regulation characteristic under the different maintenance or stock conditions.

Test 5

Multi-layer granules of the type obtained in Example 4 were used as sample. By reference to the Dissolution Test described in the Japanese Pharmacopoeia (11 version), the sample portions were placed in a revolving basket and underwent dissolution (a) with the first solvent throughout test period, (b) with the second solvent throughout test period, and (c) with the first solvent for the first 2 hours and with the second solvent for remaining hours of the test period, respectively.
The obtained results (as cumulative dilution percentages) are summarized in Table 5.

Table 5. Progress in dissolution (cumulative percentage)

| Extraction conditions | Time (hours) | | | | | | |
|---|---|---|---|---|---|---|---|
| | 1 | 2 | 3 | 4 | 6 | 8 | 10 |
| (a) | 52.1 | 62.3 | 66.1 | 72.6 | 82.4 | 88.3 | 94.4 |
| (b) | 53.1 | 62.5 | 68.3 | 73.2 | 83.9 | 87.6 | 90.8 |
| (c) | 52.7 | 62.1 | 71.4 | 79.0 | 86.5 | 92.1 | 93.6 |

Table 5 demonstrates that the effect of the membranous layer according to the present invention on the release characteristic is independent of pH.

**Claims**

**Claim for the following Contracting States : AT, BE, CH, DE, FR, GB, IT, LI, NL, SL**

1. A multi-layered granule comprising an inner, slow-releasing layer, an outer, rapid-releasing layer and an intermediate layer provided between said slow-releasing layer and said rapid-releasing layer which intermediate layer comprises 20-90% by weight of a hardened oil and 1-10% by weight of hydroxypropyl cellulose or methyl cellulose.

**Claim for the following Contracting State : ES**

1. A method for preparing a multi-layered granule, comprising an inner, slow-realeasing layer, an outer, rapid-releasing layer and an intermediate layer provided between said slow-releasing layer and said rapid-releasing layer which intermediate layer comprises 20-90% by weight of a hardened oil and 1-10% by weight of hydroxypropyl cellulose or methyl cellulose which includes coating the slow-release layer with a membranous layer to be the intermediate layer by spraying a liquid preparation to be formed into the membranous layer while the subjects to be coated are flowing and rolling, and forming a rapid-release layer over the resulting granules having the thus-formed membranous layer by the same method as in forming the intermediate membranous layer.

**Patentansprüche**

**Patentanspruch für folgende Vertragsstaaten : AT, BE, CH, DE, FR, GB, IT, LI, NL**

1. Vielschicht-Körnchen, umfassend eine innere, langsam freilassende Schicht, eine äussere, schnell freilassende Schicht und eine Zwischenschicht, die zwischen der langsam freilassenden Schicht und der schnell freilassenden Schicht angeordnet ist, wobei die Zwischenschicht 20 bis 90 Gew.% eines gehärteten Öls und 1 bis 10 Gew.% Hydroxypropylzellulose oder Methylzellulose umfasst.

**Patentanspruch für folgende Vertragsstaat : ES**

1. Verfahren zur Herstellung eines Vielschicht-Körnchens, umfassend eine innere, langsam freilassende Schicht, eine äussere, schnell freilassende Schicht und eine Zwischenschicht, die zwischen der langsam freilassenden Schicht und der schnell freilassenden Schicht angeordnet ist, wobei die Zwischenschicht 20 bis 90 Gew.% eines gehärteten Öls und 1 bis 10 Gew.% Hydroxypropylzellulose oder Methylzellulose umfasst, umfassend das Beschichten der langsam freilassenden Schicht mit einer Membranschicht, die die Zwischenschicht sein soll, durch Sprühen eines flüssigen Ansatzes, aus dem die Membranschicht gebildet werden soll, während die zu beschichtenden Subjekte fliessen und umgewälzt werden, und Bilden einer schnell freilassenden Schicht über den resultierenden Körnchen mit der so gebildeten Membranschicht durch das gleiche Verfahren wie die Bildung der Membranzwischenschicht.

**Revendications**

**Revendication pour les Etats contractants suivants : AT, BE, CH, DE, FR, GB, IT, LI, NL**

1. Granule multicouche comprenant une couche intérieure à libération lente, une couche extérieure à libération rapide et une couche intermédiaire disposée entre ladite couche à libération lente et ladite couche à libération rapide, la couche intermédiaire comprenant 20 à 90 % en poids d'une huile hydrogénée et 1 à 10 % en poids d'hydroxypropylcellulose ou de méthylcellulose.

**Revendication pour l'Etat contractant suivant : ES**

1. Procédé pour préparer un granule multicouche comprenant une couche intérieure à libération lente, une couche extérieure à libération rapide et une couche intermédiaire disposée entre ladite couche à libération lente et ladite couche à libération rapide, la couche intermédiaire comprenant 20 à 90 % en poids d'une huile hydrogénée et 1 à 10 % en poids d'hydroxypropylcellulose ou de méthylcellulose, dans lequel on enrobe la couche à libération lente d'une couche membraneuse devant constituer la couche intermédiaire en pulvérisant une préparation liquide devant être transformée en la couche membraneuse tandis que les granules à enrober s'écoulent et roulent, et on forme une couche à libération rapide sur les granules résultants comportant la couche membraneuse ainsi formée, par la même méthode que dans la formation de la couche membraneuse intermédiaire.